# EUROPÄISCHE PATENTSCHRIFT

(11) **EP 0 858 788 B1**
(45) Veröffentlichungstag und Bekanntmachung des Hinweises auf die Patenterteilung: **19.05.2004**
(21) Anmeldenummer: 98101679.3
(22) Anmeldetag: 31.01.1998
(51) Int. Cl.: A61F 9/007, A61F 9/008

(54) **Medizinisches Gerät, insbesondere zur Senkung des Augeninnendrucks**
Medical device, especially for reducing the intraocular pressure
Dispositif médical, particulièrement pour diminuer la tension intraoculaire

(30) Priorität: 15.02.1997 DE 19705815
(43) Veröffentlichungstag der Anmeldung: 19.08.1998
(73) Patentinhaber: Heidelberg Engineering Optische Messsysteme GmbH, 69121 Heidelberg (DE)
(72) Erfinder: Burk, Reinhard O.W., Dr., 69118 Heidelberg (DE)
(74) Vertreter: Schmitt, Meinrad, Dipl.-Ing.

(56) Entgegenhaltungen:
- WO-A-95/34247
- US-A- 5 222 952
- US-A- 5 370 641
- US-A- 5 431 646

## Beschreibung

Die Erfindung betrifft ein medizinisches Gerät gemäß der im Oberbegriff des Patentanspruchs 1 angegebenen Merkmale.

Ein derartiges medizinisches Gerät ist aus der US 5 431 646 bekannt und enthält einen mit einer Oberflächenbeschichtung versehenen Lichtleiter. Der Lichtleiter der Sonde ist in einem zylindrischen Quarzrohr angeordnet, wobei das Quarzrohr ggf. von einer teilweise halbschalenförmigen Kanüle aus rostfreiem Stahl umgeben ist. Das freie Ende des Lichtleiters ist abgeschrägt, wobei an der somit gebildeten Schrägfläche die Oberflächenbeschichtung eine Öffnung aufweist. An der Schrägfläche wird das Licht des Lichtleiters reflektiert und an der gegenüberliegenden Seite durch die dort vorhandene lichtdurchlässige Oberflächenbeschichtung ausgestrahlt.

Ferner ist aus der WO 92/17138 A2 ein medizinisches Gerät mit einem Laser bekannt, an welchem eine Sonde über einen Lichtleiter angeschlossen ist. Das Ende des Lichtleiterkabels oder der Lichtleiterfasern ist in einer Hülse angeordnet, welche in ein Sondengehäuse eingeführt ist, und innerhalb des Sondengehäuses sind zusätzliche lichtleitende Mittel wie Linsen und Prismen zur Strahlenleitung bzw. Strahlenumlenkung vorgesehen. Das Gerät und insbesondere die Sonde ist im Hinblick auf die recht großen Abmessungen nicht ohne weiteres in der Mikrochirurgie oder der Augenheilkunde einsetzbar.

Des weiteren sind aus der DE 38 31 141 A1 ein Verfahren und eine Vorrichtung zur Mikrochirurgie am Auge mittels Laserstrahlung bekannt. Die Vorrichtung enthält ein Handstück, in welchem zum einen ein Lichtleiter oder eine Lichtleitfaser und zum anderen eine Saug- bzw. Spüleinrichtung zur Entfernung des von der Laserstrahlung abgetragenen Gewebes vorgesehen ist. Das Licht tritt frontal aus dem freien Ende des Lichtleiters heraus, wobei das Lichtleiterende zusammen mit der Saug- bzw. Spüleinrichtung in einem besonderen Kopfteil angeordnet sind. Auch diese Vorrichtung weist nicht unerhebliche Abmessungen auf, durch welche der Einsatz in der Mikrochirurgie auf besondere Fälle beschränkt ist.

Glaukome zählen in den Industriestaaten zu den häufigsten Ursachen für eine Erblindung. Das gemeinsame Merkmal dieser Krankheitsgruppe ist in der überwiegenden Anzahl der Fälle eine Erhöhung des Augeninnendrucks über ein von Sehnerv und Nervenfaserschicht toleriertes Niveau hinaus. Unbehandelt kommt es zu einer fortschreitenden Atrophie des Sehnerven. Der progrediente Verlust von Nervenfasern führt im Spätstadium der Erkrankung zu fortschreitenden Gesichtsfeldausfällen, unbehandelt kommt es zum irreversiblen vollständigen Funktionsverlust mit Erblindung. Die Behandlung richtet sich nach der jeweiligen Form des Glaukoms, im Vordergrund steht die Senkung des intraokularen Druckes. Ursache für die Drucksteigerung ist ein erhöhter Widerstand im Ablußsystem des Kammerwassers. Das Kammerwasser, das mit einer Rate von ungefähr 2.5µl/min. gebildet wird, verläßt das Auge zu >80% im Bereich des Kammerwinkels und zu >20% im Bereich des Ziliarkörpers. Über das in der Kammerwinkelregion gelegene Trabekelwerk erreicht es den Schlemm-Kanal und wird von hier über die Kammerwasservenen unter die Bindehaut geleitet, wo die Resorption in den Bindehautgefäßen erfolgt. Der Hauptwiderstand des Kammerwasserabflusses liegt in der inneren Wand des Schlemm-Kanals und dem benachbarten Trabekelwerk. Die Behandlung der Glaukome ist im Anfangsstadium der Erkrankung zumeist medikamentös. Wenn sich kein ausreichender drucksenkender Effekt mehr erzielen läßt, kommen chirurgische oder laserchirurgische Verfahren zur Anwendung. Die bisher verfügbaren Methoden zur nichtmedikamentösen Augeninnendrucksenkung zeichnen sich dadurch aus, daß sie nicht primär am Ort der stärksten Widerstandserhöhung wirken.

Es ist die Argonlasertrabekuloplastik bekannt, bei der über ein Kontaktglas mit speziellen Linsensystemen an der Spaltlampe Laserimpulse durch die Augenvorderkammer auf das Trabekelmaschenwerk gerichtet werden, die eine Straffung des Trabekelwerkes zum Ziel haben und dadurch den Kammerwasserabfluß in den Schlemm-Kanal hinein verbessern sollen. Dieses Verfahren führt zwar zu einer vorübergehenden Augeninnendrucksenkung in den meisten Fällen, durch die Auslenkung des Laserstrahls durch die Vorderkammer hindurch auf die gegenüberliegende Seite des Maschenwerkes ist die Zielgenauigkeit jedoch begrenzt, die Ausbildung von Verklebungen (Synechien) im Bereich der Kammerwinkelregion ist eine der Komplikationen. Insbesondere ist die Eindringtiefe der Laserimpulse schwer zu kontrollieren.

Bekannt ist auch die Photoablation des Trabekelwerks ab interno, bei der als Laserquellen entweder ein Erbium-Yag Laser (Er:Yag) oder ein Neodymium-Yag Laser (Nd:Yag) oder ein Excimer-Laser eingesetzt werden, deren Impulse mittels Fiberoptiken durch die Augenvorderkammer hindurch auf das jeweils gegenüberliegende Trabekelmaschenwerk gerichtet sind. Darüber hinaus wurden Versuche unternommen, die Laserimpulse eines Nd:Yag Lasers durch ein Kontaktglas auf die gegenüberliegende Kammerwinkelregion zu applizieren, ohne das Auge zu eröffnen. Alternativ wurden ab interno Sklerostomien experimentell auch mit gepulstem Farbstofflaser oder Argon-Laser über Kontaktglas durchgeführt. Auch bei diesen Verfahren ist die Eindringtiefe der Laserimpulse schwer zu kontrollieren. Viele Experimentatoren versuchen, einen durchgreifenden Effekt durch die angrenzende Lederhaut hindurch zu erzielen, um den Subkonjunktivalraum (Region unmittelbar unter der Augenapfelbindehaut) zu eröffnen und einen direkten Abfluß in dieses Gebiet zu ermöglichen. Aufgrund der Narbenreaktionen haben die erwähnten Techniken keine Verbreitung über einen klinisch experimentellen Ansatz hinaus erlangen können.

Bei der bekannten Laser-Sklerostomie ab externo wird eine Fistel zum Kammerwasseraustritt unter die Bindehaut geschaffen, wobei mittels eines Excimer-Lasers oder eines Er:Yag Lasers nach Eröffnung der Augenbindehaut die Sklera in gesamter Dicke durchschossen wird. Bei diesem Verfahren resultiert ein durchgreifender Defekt in der Augenlederhaut (Sklera). Die dauerhafte Resorption des Kammerwassers setzt voraus, daß der Zugang nicht vernarbt, was bei diesem Verfahren jedoch eine typische Komplikation darstellt, ebenso wie bei den herkömmlich manuell durchgeführten Operationen. Im Gegensatz zu den konventionellen chirurgischen Verfahren wird die Iris (Regenbogenhaut) bei der Laser Sklerostomie jedoch nicht zusätzlich partiell entfernt (Iridektomie), wodurch sich als weitere Komplikationsmöglichkeit der Verschluß der geschaffenen Fistel durch das Hineinziehen der Iris ergibt. Durch den zusätzlichen medikamentösen Einsatz von Antimetaboliten wie Mitomycin C oder 5-Fluorouracil wird oftmals versucht, die überschießende Vernarbung im Bereich der Bindehaut zu kontrollieren. Als gravierender Nachteil ist hier die hohe Toxizität der verwendeten Substanzen anzuführen.

Allen derzeit im klinischen Einsatz oder in Laborversuchen experimentell erprobten medizinischen Geräten und Verfahren ist gemeinsam, daß sie nicht selektiv eine Verbesserung des Kammerwasserabflusses an den intraokularen Strukturen erreichen, die den Ort des Hauptwiderstandes im Ausflußssystem aus der Augenvorderkammer darstellen. Bei den bekannten Verfahren wird der Laserimpuls durch die Vorderkammer hindurch auf gegenüberliegende Gewebeariale gerichtet. Bei den bekannten Verfahren erfolgen die ersten Effekte im Bereich des Trabekelmaschenwerks, wobei die in den tieferen Strukturen gleichfalls auftretenden Wirkungen nicht abgeschätzt werden können.

Der Erfindung liegt die Aufgabe zugrunde ein medizinisches Gerät zu schaffen, welches die aufgezeigten Nachteil vermeidet und bei kompakter kleiner Bauweise gleichwohl eine einfache Handhabung ermöglicht. Das medizinische Gerät soll vor allem in der Mikrochirurgie und/oder in der Augenheilkunde zum Einsatz gelangen. Mit dem medizinischen Gerät soll vor allem der Abflußwiderstand für das Kammerwasser im Augeninneren an genau den Strukturen reduziert werden, die für den Hauptbestandteil des intraokularen Kammerwasser-Abflußwiderstandes verantwortlich sind und die übrigen Gewebebestandteile geschont werden.

Diese Aufgabe wird gemäß Merkmalen des Patentanspruchs 1 gelöst. Vorteilhafte Ausgestaltungen der Erfindung sind in den Unteransprüchen angegeben.

Das erfindungsgemäße Gerät zeichnet sich durch eine funktionsgerechte Konstruktion und einem kompakten Aufbau aus. Es ist in der Mikrochirurgie zur Abtragung oder Veränderung von Gewebe und insbesondere in der Augenheilkunde zur Reduktion des Augeninnendrucks geeignet. Das Gerät ermöglicht im Gegensatz zu den bekannten laserchirurgischen Ansätzen eine der Abflußrichtung einer Gewebsflüssigkeit entgegengesetzte Reihenfolge der Gewebeabtragung. So läßt sich mittels des erfindungsgemäßen Geräts eine Schlemm-Kanal Laser-Trabekulotomie (SKLT) durchführen, die darauf abzielt, den Abflußwiderstand im Augeninneren an genau den Strukturen zu reduzieren, die für den Hauptbestandteil des intraokularen Kammerwasserabflußwiderstandes verantwortlich sind. Alle übrigen Gewebebestandteile sollen so weit wie irgend möglich geschont werden. Das medizinische Gerät ermöglicht die Senkung des Augeninnendrucks durch Bearbeiten des Schlemmkanals und/oder des dem Schlemmkanal unmittelbar angelagerten Gewebes mittels der Lichtleitersonde mit einem gebogenen und/oder flexiblen Abschnitt. Mittels des erfindungsgemäßen Geräts wird in besonders zweckmäßiger Weise die Erzeugung von Poren in Gewebe, insbesondere im Trabekelmaschenwerk ermöglicht, ohne daß benachbarte Gewebestrukturen in Mitleidenschaft gezogen werden. Dies gilt ganz allgemein in der Mikrochirurgie und Gewebebehandlung.

In der Augenheilkunde wird das Besondere an dem mittels des erfindungsgemäßen Gerätes durchführbaren Verfahren darin gesehen, daß im Gegensatz zu den bislang in der Erprobung befindlichen laserchirurgischen Ansätzen die zur Abflußrichtung des Kammerwassers entgegengesetze Reihenfolge der Gewebeabtragung erfolgt. Der Kammerwasserstrom verläßt das Auge, indem er aus der Vorderkammer über das Trabekelmaschenwerk durch die Innenwand des Schlemm-Kanals in den Schlemm-Kanal eintritt und von hier aus über die Kammerwasservenen das Auge verläßt. In dieser identischen Reihenfolge wird die Gewebeabtragung bei bisher in Erprobung befindlichen minimal invasiven laserchirurgischen ab interno Techniken herbeigeführt. Hierbei kann jedoch die Eindringtiefe nur schlecht gesteuert werden, eine Eröffnung der Außenwand des Schlemm-Kanals ist nicht sicher auszuschließen. Von einigen Arbeitsgruppen wird dies sogar bewußt herbeigeführt und die benachbarte Augenlederhaut zusätzlich durchdrungen, um zusätzlich einen Kammerwasserabfluß unter die Bindehaut des Augapfels zu erreichen. Die Schlemm-Kanal Laser Trabekulotomie hat dagegen zum Ziel, den Schlemm-Kanal selbst weitestgehend intakt zu lassen und lediglich Poren in der Innenwand, die der Augenvorderkammer benachbart ist und nur durch das Trabekelwerk von dieser getrennt wird, zu schaffen. Die bei der Schlemm-Kanal Laser Trabekulotomie zu behandelnden Strukturen sind die Innenwand des Schlemm-Kanals (der Vorderkammer zugewandte Begrenzung des Schlemm-Kanals) und das juxtakanalikuläre (dem Schlemm-Kanal unmittelbar angelagertes) Gewebe des Trabekelmaschenwerks. Es handelt sich erfindungsgemäß bei der SKLT um die Kombination der bekannten laserinduzierten Gewebeabtragung aus dem Bereich des Trabekelmaschenwerkes mit dem bekannten Operationszugang der chirurgischen Trabekulotomie nach Harms und Mackensen.

Die Erfindung wird in der folgendenden Beschreibung anhand der in der Zeichnung dargestellten Ausführungsbeispiele näher erläutert, ohne daß insoweit eine Beschränkung erfolgt. Es zeigen:
- Fig. 1: eine erste Ausführungsform für die Ausgestaltung eines derartigen Gerätes,
- Fig. 2: eine weitere Ausführungsform des Gerätes gemäß Fig. 1,
- Fig. 3: eine Ausgestaltung des Lichtleiters als nahezu geschlossener Kreisring,
- Fig. 4: eine Ausgestaltung der Ausführungsform gemäß Fig. 3.

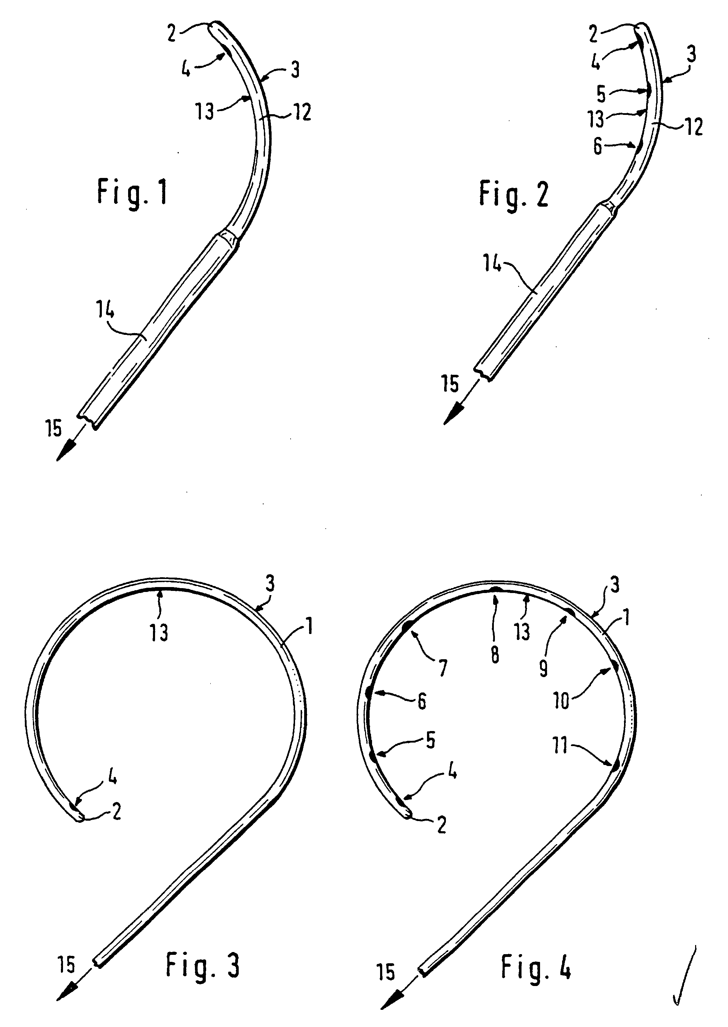

Mit dem erfindungsgemäßen Gerät wird nach der chirurgischen Präparation des Zuganges zum Schlemm-Kanal anstelle der herkömmlichen chirurgischen Trabekulotomiesonde, mit der lediglich ein ungezieltes und weitgehend unkontrolliertes Aufreißen der Gewebestrukturen vorgenommen werden kann, ein flexibler Lichtleiter 12 bzw. ein Lichtleiter, der aus einem elastischen und/oder plastischen Material besteht, in den Schlemm-Kanal eingeführt. Der Schlemm-Kanal stellt eine ringförmige Struktur dar, der Kreisdurchmesser des Schlemm-Kanals ist geringfügig größer als der Hornhautdurchmesser des jeweils zu behandelnden Patienten. Für die Einführung in den Schlemm-Kanal von Augen mit einem horizontalen Hornhautdurchmesser von 10 bis 12 mm ist die Lichtleitersonde 12 in Richtung der Krümmungsinnenseite 13 flexibel. Der Sondendurchmesser beträgt maximal 400 µm, insbesondere maximal 320 µm, um atraumatisch in einen normalen Schlemm-Kanal eingeführt werden zu können. Da einige wenige Augen Schlemm-Kanalweiten von lediglich 200µm Durchmesser aufweisen, können kleinere Lichtleiterdurchmesser ebenfalls zur Anwendung kommen. Bei dem in den Fig. 1 und 2 dargestellten Ausführungsbeispiel erstreckt sich der Lichtleiter 12 über einen Kreisbogen von etwa 90° bis 120° und ist für das Einführen von rechts oder links in den Schlemm-Kanal rechts- und linksläufig. Der Krümmungs- oder Biegeradius liegt im Größenbereich von einigen Millimetern und ist insbesondere größer als 4 mm, vorzugsweise größer als 5 mm; der genannte Radius beträgt maximal 7 mm, vorzugsweise 6 mm. Die Lichtleiterspitze 2 ist erfindungsgemäß abgerundet.

Die Sonde weist eine Oberflächenbeschichtung 3 auf, die den Austritt des Laserstrahls an einer Austrittsöffnung 4 (Fig.1), die sich in der Nähe der Sondenspitze 2 befindet, bzw. mehreren Austrittsöffnungen 4 bis 6 (Fig. 2) in einem Winkel von 90° in Richtung der Innenkrümmung 13 des flexiblen Lichtleiters 12 sicherstellt. Als Oberflächenbeschichtung 3 verwendet man eine die entsprechende Wellenlänge absorbierende Farbbeschichtung bzw. Verspiegelung oder anderweitige Abschirmungen, z.B. aus Carbonmaterial oder Kunststoff. An den kreisbogenförmigen Abschnitt des Lichtleiters 12 schließt ein Handgriff 14 an, in den der Lichtleiter 12 eingebettet ist. Der Handgriff 14 kann auch aus der von der Sonde vorgegebenen Krümmung um etwa 90° herausragen. Der Lichtleiter besitzt einen einen Laseranschluß 15. Erfindungsgemäß ist der Lichtleiter in seinem die Sonde bildenden Endbereich zumindest teilweise flexibel ausgebildet und mit der für Licht bzw. die Laserstrahlen undurchlässigen Oberflächenbeschichtung 3 versehen. Diese Oberflächenbeschichtung 3 besitzt wenigstens eine Austrittsöffnung, durch welche das Licht bzw. die Laserstrahlen zur Seite aus dem Lichtleiter austreten können. Der an den Laser angeschlossene Lichtleiter ist elastisch und/oder plastisch zu einem gebogenen Abschnitt verformt oder verformbar. Auch wenn der flexible und/oder biegbare und/oder gebogene Abschnitt des mit der Oberflächenbeschichtung versehenen Abschnitts zweckmäßig näherungsweise einen Kreisbogen bildet, so können auch andere Kurvenformen, wie eine Parabel, Ellipse oder dergleichen vorgegeben oder erreicht werden, um für den jeweiligen Einsatz des erfindungsgemäßen medizinischen Geräts die optimalen Voraussetzungen zu gewährleisten. Unabhängig von der jeweiligen konkreten Ausbildung der Biegung liegt die wenigstens eine Austrittsöffnung bevorzugt im Bereich der Innenseite des genannten Abschnitts. An den flexiblen und/oder biegbaren und/oder gebogenen Abschnitt schließt erfindungsgemäß der Handgriff 14 an, durch welchen der Lichtleiter 12 hindurchgeführt ist. Das andere Ende des Lichtleiters 12 ist mit dem erwähnten Laseranschluß 15 verbunden. Die Spitze 2 ist bevorzugt mit der erfindungswesentlichen Oberflächenbeschichtung versehen, so daß dort in Längsrichtung in zweckmäßiger Weise kein Laserlicht austreten kann.

Diese Sonde ist an einen handelsüblichen Er:Yag Laser anschließbar. Bei einer Energie von 4mJ können bei einer Impulsdauer von 100 Mikrosekunden, 150 Mikrosekunden und 250 Mikrosekunden im Trabekelmaschenwerk durchgreifende Gewebeabtragungen mit Porengrößen von circa 100µm, 120µm und 200µm erzeugt werden. Die thermischen Effekte im Porenrandbereich liegen in der Größenordnung von bis zu 10µm, 20µm und bis zu 50µm. Um mehrere Poren innerhalb der Innenwand des Schlemm-Kanals zu erzielen, wird die Sonde 12 in den Schlemm-Kanal eingeführt.

Bei den Ausführungsformen nach den Fig. 3 und 4 erstreckt sich ein derartiger Lichtleiter 1 in einer nahezu vollständigen Kreisform und kann somit den gesamten Schlemm-Kanal erfassen. Gemäß Fig. 3 befindet sich eine Austrittsöffnung 4 im Bereich der Sondenspitze 2.

Neben der beschriebenen einzelnen Laserstrahlaustrittsöffnung 4 in der Nähe der Lichtleiterspitze 2 können mehrere Stellen 5 bis 11 gleichzeitig beispielsweise im Abstand von 100°, 60° oder 40° für beispielsweise drei, fünf oder acht simultane Laserimpulse am Innenradius des Lichtleiters freigegeben sein (Fig. 4).

Als Laserquellen können auch ein Holmium Laser oder ein Nd:YLF-Pikosekunden-Lasersystem zur Anwendung kommen. Bei Verwendung eines Nd:YLF-Lasersystems kann die zur Gewebeablation erforderliche Energie weiter reduziert werden. Denkbar ist die Benutzung eines zweistufigen Systems, das aus einem diodengepumpten, aktiv modemgekoppelten Oszillator und einem regenerativen Verstärker besteht. Die im Oszillator bei einer Wellenlänge von 1053 nm erzeugten Pulse haben eine Länge von ca. 25ps bei einer Pulsenergie von 0,2nJ. Über eine Polarisationsschaltung werden diese Pulse in den regenerativen Verstärker eingekoppelt und dort bis zu einer Pulsenergie von 1,5 mJ verstärkt. Bei Pulsdauern von ca 30ps tritt der Prozeß der Gewebeabtragung bereits bei Energiedichten von 20J/cm² ein. Diese geringen Energien erlauben eine nahezu lokale Gewebeabtragung mit minimaler schädigender Auswirkung auf umliegende Strukturen.

Des weiteren ist die Verwendung eines komerziell erhältlichen Diodenlasersystems in Zusammenhang mit dem geschilderten erfindungsgemäßen Verfahren und Gerät ohne weiteres möglich, insbesondere wenn aufgrund spezieller anatomischer Gegebenheiten ein Koagulationseffekt gewünscht ist. Die Bezeichnung für diese Variante der SKLT ist die Schlemm-Kanal Laser Trabekulo-Koagulation.

Die Gewebeabtragung erfolgt entgegengesezt zur Kammerwasserabflußrichtung. Hierdurch wird sichergestellt, daß lediglich die Regionen, die die Bereiche des größten Widerstandes im Kammerwasserabflußsystem darstellen, therapiert werden.

Das Verfahren der Schlemm-Kanal Laser Trabekulotomie ist nach folgenden Kriterien konzipiert worden:
Durch die Einführung der lichtleitenden Faser in den Schlemm-Kanal werden als Haupteffekte erreicht, daß
   (1) der gewebeabtragende Laserstrahl direkt auf die Innenwand des Schlemm-Kanals gerichtet ist und damit unmittelbar in Nähe der zu behandelnden Struktur liegt. Es wird sichergestellt, daß die Innenwand des Schlemm-Kanals behandelt wird und auch das angrenzende (juxtakanalikuläre) Trabekelmaschenwerk laserchirurgisch bearbeitet werden kann;
   (2) der Außenradius des Lichtleiters an der äußeren Wand des Schlemm-Kanals entlangläuft und diese Wandstruktur somit gleichzeitig vor unerwünschten Effekten mechanisch geschützt ist.

Diese Vorgehensweise ermöglicht es Poren im Trabekelmaschenwerk zu erzeugen, ohne benachbarte Strukturen in Mitleidenschaft zu ziehen.

Im Gegensatz zu dem hier dargelegten Verfahren ist es bisher üblich, den Laserimpuls durch die Vorderkammer hindurch auf gegenüberliegende Gewebeareale zu richten. Die ersten Effekte erfolgen bei den bereits bekannten Verfahren im Bereich des Trabekelmaschenwerkes, die in tieferen Strukturen ebenfalls auftretenden Wirkungen können nicht abgeschätzt werden.

## Patentansprüche

1. Medizinisches Gerät, insbesondere zur Augeninnendrucksenkung durch Bearbeiten des Schlemm-Kanals und des dem Schlemm-Kanal unmittelbar angelagerten Gewebes des Trabekelmaschenwerkes, enthaltend eine Sonde mit einem an einen Laser anschließbaren Lichtleiter (1, 12), welcher mit einer, wenigstens eine Öffnung (4, 5 bis 11) aufweisenden Oberflächenbeschichtung (3) versehen ist,
**dadurch gekennzeichnet, dass** die Sonde als der Endabschnitt des Lichtleiters ausgebildet ist, dass die Lichtleitersonde (1, 12) in Richtung zu einer Krümmungsinnenseite (13) gebogen oder biegbar ausgebildet ist, dass die Oberflächenbeschichtung (3) der Lichtleitersonde (1, 12) für Laserlicht undurchlässig ausgebildet ist und dass die wenigstens eine Öffnung als Austrittsöffnung (4, 5 bis 11) für das Laserlicht ausgebildet und im Bereich der Krümmungsinnenseite (13) angeordnet ist.

2. Medizinisches Gerät nach Anspruch 1, **dadurch gekennzeichnet, daß** die wenigstens eine Austrittsöffnung (4, 5 bis 11) den Austritt des Laserlichts in einem Winkel von im wesentlichen 90° zum Verlauf oder der Tangente der Lichtleitersonde (1, 12) ermöglicht.

3. Gerät nach Anspruch 1 oder 2, **dadurch gekennzeichnet, daß** die wenigstens eine Austrittsöffnung (4) im Bereich der Lichtleiterspitze (2) vorgesehen ist.

4. Gerät nach einem der Ansprüche 1 bis 3, **dadurch gekennzeichnet, daß** die Lichtleitersonde (1, 12) gemeinsam mit der die Austrittsöffnung (4, 5 bis 11) aufweisenden Oberflächenbeschichtung (3) flexibel ausgebildet ist und/oder elastisch und/oder plastisch verformbar ausgebildet ist.

5. Gerät nach einem der Ansprüche 1 bis 4, **dadurch gekennzeichnet, daß** sich die Lichtleitersonde (12) über eine Bogenlänge, insbesondere eine Kreisbogenlänge, in der Größenordnung von 90° bis 120° erstreckt und entweder rechts- oder linksläufig geformt ist.

6. Gerät nach einem der Ansprüche 1 bis 5, **dadurch gekennzeichnet, daß** sich die Lichtleitersonde (1) über einen gesamten Bogen, insbesondere Kreisbogen des Schlemm-Kanals, erstreckt.

7. Gerät nach einem der Ansprüche 1 bis 6, **dadurch gekennzeichnet, daß** die Lichtleitersonde (1, 12) eine abgerundete Lichtleiterspitze (2) aufweist und/oder daß beabstandet zur Lichtleiterspitze (2) ein Handgriff (14) vorgesehen ist, durch welchen die Lichtleitersonde (1, 12) insbesondere zu einem Anschluß (15), geführt ist.

8. Gerät nach einem der Ansprüche 1 bis 7, **dadurch gekennzeichnet, daß** die Lichtleitersonde (1, 12) zur Abgabe von simultanen Laserimpulsen zur Krümmungsinnenseite (13) gerichtet mehrere Austrittsöffnungen (4 bis 11) aufweist.

9. Gerät nach einem der Ansprüche 1 bis 8, **dadurch gekennzeichnet, daß** an die Lichtleitersonde (1) ein Er:Yag Laser angeschlossen ist oder daß als Laserquellen ein Holmium Laser oder ein Nd:YLF-Lasersystem vorgesehen sind oder daß ein Diodenlasersystem vorgesehen ist.

10. Gerät nach einem der Ansprüche 1 bis 9, **dadurch gekennzeichnet, daß** der Durchmesser der Lichtleitersonde (1, 12) mit der Oberflächenbeschichtung (3) maximal 400 µm groß ist, und insbesondere maximal 320 µm groß ist und/oder daß der Biegeradius der Lichtleitersonde (1, 12) in der Größenordnung von wenigstens 4 mm, vorzugsweise wenigstens 5 mm liegt und/oder maximal in der Größenordnung von 7 mm, insbesondere maximal 6 mm, liegt.

## Claims

1. Medical apparatus, in particular for reducing intraocular pressure by acting on the Schlemm's canal and tissue of a trabecular network directly in contact with the Schlemm's canal, the apparatus comprising a probe with an optical fibre (1, 12) connectable to a laser, which is provided with a surface coating (3) having at least one aperture (4, 5 to 11), **characterised in that** the probe is designed as the end portion of the optical fibre, **in that** the optical fibre probe (1, 12) is bent, or designed to be bent, in the direction of the inner side of a curve (13), **in that** the surface coating (3) of the optical fibre probe (1, 12) is designed to be impermeable to laser light and **in that** the at least one aperture is designed as an emission aperture (4, 5 to 11) for the laser light and is arranged in the region of the inner side of a curve (13).

2. Medical apparatus according to claim 1, **characterised in that** the at least one emission aperture (4, 5 to 11) permits laser light to issue at an angle of substantially 90° to the course or tangent of the optical fibre probe (1, 12).

3. Apparatus according to claim 1 or 2, **characterised in that** the at least one emission aperture (4) is provided in the region of the tip of the optical fibre (2).

4. Apparatus according to any of claims 1 to 3, **characterised in that** the optical fibre probe (1, 12) together with the surface coating (3) comprising the emission aperture (4, 5 to 11) are flexible and/or elastically and/or plastically deformable.

5. Apparatus according to any of claims 1 to 4, **characterised in that** the optical fibre probe (12) extends over an arc length, in particular an arc-of-a-circle length, of about 90° to about 120° and is formed left to right or right to left.

6. Apparatus according to any of claims 1 to 5, **characterised in that** the optical fibre probe (1) extends over an entire arc, in particular an arc of a circle, of the Schlemm's canal.

7. Apparatus according to any of claims 1 to 6, **characterised in that** the optical fibre probe (1, 12) has a rounded tip (2) and/or **in that** a handle (14) spaced a distance from the optical fibre tip (2) is provided, through which the optical fibre probe (1, 12) is guided, in particular to a terminal (15).

8. Apparatus according to any of claims 1 to 7, **characterised in that** the optical fibre probe (1, 12) has a plurality of emission apertures (4 to 11) facing the inner side of a curve (13) to issue simultaneous laser pulses.

9. Apparatus according to any of claims 1 to 8, **characterised in that** an Er:Yag laser is connected to the optical fibre probe (1), or **in that** a holmium laser or an Nd:YLF laser is provided as the laser sources, or **in that** a diode laser system is provided.

10. Apparatus according to any of claims 1 to 9, **characterised in that** the diameter of the optical fibre probe (1, 12) and surface coating (3) combined is at most 400 µm, in particular 320 µm, and/or **in that** the radius of curvature of the optical fibre probe (1, 12) is approximately at least 4 mm, preferably at least 5 mm and/or is at most approximately 7 mm, in particular at most 6 mm.

## Revendications

1. Instrument médical, en particulier pour faire baisser la pression intraoculaire par traitement du canal de Schlemm et du tissu du réseau de trabécules placé directement sur le canal de Schlemm, comprenant une sonde avec un conducteur optique (1, 12) pouvant se raccorder à un laser, conducteur qui est muni d'un revêtement de surface (3) présentant au moins une ouverture (4, 5 à 11), ***caractérisé en ce que*** la sonde est conformée en section d'extrémité du conducteur optique, ***en ce que*** la sonde conductrice optique (1, 12) est réalisée cintrée ou cintrable en direction d'un côté intérieur (13) de courbure, ***en ce que*** le revêtement de surface (3) de la sonde conductrice optique (1, 12) est réalisé transparent à la lumière laser et ***en ce que*** l'ouverture au nombre d'au moins une est conformée en ouverture de sortie (4, 5 à 11) pour la lumière laser et est placée au niveau du côté intérieur (13) de courbure .

2. Instrument médical selon la revendication 1, ***caractérisé en ce que*** l'ouverture de sortie (4, 5 à 11) au nombre d'au moins une permet la sortie de la lumière laser selon un angle de sensiblement 90° par rapport au parcours ou à la tangente de la sonde conductrice optique (1, 12).

3. Instrument selon la revendication 1 ou 2, ***caractérisé en ce que*** l'ouverture de sortie (4) au nombre d'au moins une est prévue au niveau de la pointe (2) du conducteur optique.

4. Instrument selon l'une quelconque des revendications 1 à 3, ***caractérisé en ce que*** la sonde conductrice optique (1, 12) est réalisée flexible en commun avec le revêtement de surface (3) présentant l'ouverture de sortie (4, 5 à 11), et/ou est conformée déformable élastiquement et/ou plastiquement.

5. Instrument selon l'une quelconque des revendications 1 à 4, ***caractérisé en ce que*** la sonde conductrice optique (12) s'étend sur une longueur d'arc, en particulier une longueur d'arc de cercle, de l'ordre de 90° à 120° et est formée tournant à droite ou à gauche.

6. Instrument selon l'une quelconque des revendications 1 à 5, ***caractérisé en ce que*** la sonde conductrice optique (1) s'étend sur un arc complet, en particulier un arc de cercle du canal de Schlemm.

7. Instrument selon l'une quelconque des revendications 1 à 6, ***caractérisé en ce que*** la sonde conductrice optique (1, 12) présente une extrémité (2) de conducteur optique arrondie, et/ou ***en ce qu***'à distance de la pointe (2) du conducteur optique est prévue une poignée (14) à travers laquelle la sonde conductrice optique (1, 12) passe, en particulier en direction d'un raccordement.

8. Instrument selon l'une quelconque des revendications 1 à 7, ***caractérisé en ce que*** la sonde conductrice optique (1, 12) présente plusieurs ouvertures de sortie (4 à 11) orientées en direction du côté intérieur (13) de courbure pour délivrer des impulsions laser simultanées.

9. Instrument selon l'une quelconque des revendications 1 à 8, ***caractérisé en ce qu***'un laser Er:YAG est raccordé à la sonde conductrice optique (1) ou ***en ce que,*** comme source laser, un laser à holmium ou un système laser Nd :YLF est prévu, ou ***en ce qu***'un système de laser à diode est prévu.

10. Instrument selon l'une quelconque des revendications 1 à 9, ***caractérisé en ce que*** le diamètre de la sonde conductrice optique (1, 12) avec le revêtement de surface (3) vaut au maximum 400 µm, en particulier au maximum 320 µm, et/ou ***en ce que*** le rayon de courbure de la sonde conductrice optique (1, 12) est de l'ordre d'au moins 4 mm, de préférence au moins 5 mm, et/ou au maximum de l'ordre de 7 mm, en particulier au maximum 6 mm.
